# EUROPEAN PATENT SPECIFICATION

(11) **EP 2 680 821 B1**
(45) Date of publication and mention of the grant of the patent: **01.08.2018**
(21) Application number: 12719451.2
(22) Date of filing: 03.03.2012
(51) Int. Cl.: A61K 31/704, A61K 31/375, A61K 31/519, A61K 9/127, A61K 45/06

(54) **LIPOSOME FORMULATION COMPRISING AN ANTI-TUMOUR ACTIVE SUBSTANCE, METHOD FOR ITS PREPARATION AND PHARMACEUTICAL COMPOSITIONS COMPRISING IT**
LIPOSOMENFORMULIERUNG MIT ANTITUMORWIRKSTOFF, VERFAHREN ZU IHRER HERSTELLUNG UND DIESE ENTHALTENDE PHARMAZEUTISCHE ZUSAMMENSETZUNGEN
FORMULATION LIPOSOMALE COMPRENANT UNE SUBSTANCE ACTIVE ANTITUMORALE, PROCÉDÉ DE PRÉPARATION ET COMPOSITIONS PHARMACEUTIQUES LA COMPRENANT

(30) Priority: 03.03.2011 PL 39408211
(43) Date of publication of application: 08.01.2014
(73) Proprietor: Wroclawskie Centrum Badan EIT + SP Z O.O., 54-066 Wroclaw (PL)
(72) Inventor: GUBERNATOR, Jerzy, 50-511 Wroclaw (PL); KOZUBEK, Arkadiusz, 51-691 Wroclaw (PL); PADUSZYNSKI, Piotr, 98-346 Skomlin (PL); LIPKA, Dominik, 32-305 Olkusz (PL)
(74) Representative: Witek, Rafal
(86) International application number: PCT/IB2012/051014
(87) International publication number: WO 2012/117385

(56) References cited:
- EP-A2- 0 160 286
- EP-A2- 0 361 894
- US-A- 5 648 090
- US-A- 6 146 659
- RIVERA E: "Liposomal anthracyclines in metastatic breast cancer: Clinical update", THE ONCOLOGIST, ALPHAMED PRESS, US, vol. 8, no. SUPPL. 2, 1 August 2003 (2003-08-01), pages 3-9, XP008117303, ISSN: 1083-7159, DOI: 10.1634/THEONCOLOGIST.8-SUPPL_2-3
- PEREZ-SOLER R ET AL: "THE USE OF LIPOSOMES AS CARRIERS OF LIPOPHILIC ANTHRACYCLINE ANTIBIOTICS", JOURNAL OF LIPOSOME RESEARCH, TAYLOR & FRANCIS, PHILADELPHIA, PA, US, vol. 4, no. 1, 1 January 1994 (1994-01-01) , pages 555-573, XP000564587, ISSN: 0898-2104
- KIRBY C J ET AL: "STABILIZATION OF ASCORBIC ACID BY MICROENCAPSULATION IN LIPOSOMES", INTERNATIONAL JOURNAL OF FOOD SCIENCE AND TECHNOLOGY, BLACKWELL SCIENTIFIC PUBLICATIONS, OXFORD, GB, vol. 26, no. 5, 1 January 1991 (1991-01-01), pages 437-449, XP009045590, ISSN: 0950-5423

## Description

The subject of the present invention is a liposome formulation containing an anti-tumour active compound, a method of producing it and a pharmaceutical composition containing it, for use in medicine.

Liposomes are uni- or multilamellar closed structures in which a bilayer of an amphiphylic lipids encloses a microdrop of water (unilamellar liposomes) or else lipid membranes are formed concentrically and interspersed with aqueous layers (multilamellar liposomes). Amphiphylic lipids that form the bilayer possess a polar hydrophilic group and one or more linear hydrophobic polycarbon chains (>C8). The polar groups may be derivatives of phosphates, sulphates and nitrogen compounds, but the most commonly used ones are phospholipids, particularly those of natural origin, such as phosphatidylcholines obtained using the refinement of plant lipids, synthetic phospholipids, available commercially phospholipid formulations, including chemically modified phospholipids using derivatives of ethylene glycol and cholesterol. Depending on solubility, the medicinal substance is located in the aqueous layer or the lipid layer of the liposomes.

There are several methods for liposomes production. A classic method of producing multilayer liposomes is based on evaporating a lipid-organic solvent solution and rehydration of the lipid film with an aqueous solution of the medicinal compound (J. Mol. Biol. 13 (1965), 238-252). Other techniques encompass the emulsification of a lipid into a two-phase mixture of an aqueous and organic phase containing the lipid, with the simultaneous evaporation of the organic solvent (e.g. patent descriptions US 4,522,803, 5,030,453 and 5,169,637), formation of a water-in-oil emulsion, from which the organic phase is evaporated in order to produce a gel, which is then mixed to produce oligolamellar liposomes (US 4,235,871) as well as multiple freeze/thaw cycles (US 5,008,050). Unilamellar liposomes are obtained from multilamellar liposomes using ultrasounds, extrusion (e.g. US 4,975,282), homogenization, as well as injection of etheric or ethanol solutions of lipids into the aqueous phase (Deamer R., Uster, P. "Liposome preparation; Methods and Mechanisms", in: "Liposomes", ed. M.Ostro, Marcel Dekker, New York, 1987).

In the case of many groups of therapeutics, both the efficiency of enclosure of the active compound in vesicles as well as the stability of liposomes (*in vitro* and *in vivo*) are a serious technical problem. In particular, classical liposome formulations of highly insoluble in water taxoids, based on soya lecithin or a synthetic phospholipid analogue (Bartoli et al. J. Microencapsulation 7, 1990, 191-197, Riondel et al. In Vivo 6, 1992,23-28), exhibit a tendency to aggregate, as well as instability which causes the "leakage" of the active compound from the liposomes and its crystallization. In many cases, the manufacturing of a liposome formulations of the anti-tumour compounds with an effective lipid/drug ratio requires the use of special procedures, like for example described in the publication WO 9202208 and the application EP 546951 A1 an addition of negatively charged phospholipids, or the addition of a polyhydroxy alcohol and quarternary ammonium salts as described in the Japanese patent description JP 06254379. Improved liposome structures ensuring greater stability through the steric stabilization of the surface of the lipid bilayer are achieved by the so-called "Stealth" liposomes (D. D. Lasic, F. Martin "Stealth Liposomes", CRC Press Boca Raton, 1995). One method aimed at designing more durable forms of liposomes is the use of a hydrophilic polyethylene glycol coating, as described in the publication of international application WO 9422429. Medicine has seen the introduction of a liposome-enclosed form of doxorubicin, coated with polyethylene glycol under the trade name Doxil®. This Doxil® preparation contains doxorubicin encapsulated in Stealth liposome carriers, composed of three lipid components: hydrogenated soya lecithin, cholesterol and a carbamate conjugate of distearylphosphatidylethanolamine with a methoxyl derivative of polyethylene glycol 2000 in an appropriate molar ratio. The long half-life of Stealth liposomes, together with low drug leakage, is obtained using unique encapsulation methods, which ensure high loading efficiency and long-term drug retention. These methods encompass loading with an electrolyte gradient (application EP 361894 A1) or pH gradient (publication WO 8806442). In WO 8806442 publication, the components of the lipid layer constitute classic lipid compounds, such as natural and synthetic phosphatidylcholines, with the possible addition of cholesterol, and in the encapsulation process a pH gradient is used. According to the description, the limited leakage rate of the encapsulated biologically active compound is a result of the pH difference between the two sides of the lipid membrane. The pH gradient loading method is, however, limited solely to water phase soluble drugs, which are weak acids or bases. Liposome formulations containing topotecan and lipids in a 0.05:0.2 ratio, loaded using a pH gradient or ionophore loading method, are described in the publication WO 0202078. The lipid layer includes sphingomyelin and cholesterol. The international application WO 9915153 discloses, amongst others, taxol-containing liposomes, characterized by an active compound concentration in the liposomes no higher than 5 mg/ml and that contain the synthetic lecithin dilauroylphosphatidylcholine as the lipid. The authors declare that the drug:lipid ratio is in the range from 1:1 to 1:2000, preferably 1:30, but present no information about the stability of these liposome formulations, meant for administering the anti-tumour compounds through inhalation.

Stable liposome formulations have thus far been obtained mainly through the use of special procedures or active compound molecule modification, i.e. through the attachment of hydrocarbon, polymeric or peptide chains. In Polish patents Nos. 190077 and 190078 a high encapsulation efficiency of doxorubucin and mithoxanthrone at a preferable ratio of drug to lipid in a liposome formulation was obtained as a result of the lipid layer composition modification, which contains, in addition to classic components, egg lecithin and hydrogenated egg lecithin, a hydrogen sulphate acyl derivative of resorcine. Stable liposome formulations of paclitaxel have been obtained by the addition of cardiolipin to the lipid formulation (US patent descriptions Nos. 5,424,073, 5,648,090, 5,939,567 and 6,146,659). The description of US patent No. 6,146,659 discloses that in this case, the incorporation efficiency of paclitaxel in liposome vesicles exceeds 90%, with a mass ratio of active compound to lipid carrier of about 7%. In general, the possibility of incorporating paclitaxel into liposomes, as a result of its high hydrophobicity is limited to 1-10% (w/w), most often 2-8% (w/w) in relation to the lipid carrier. This coefficient can be improved only slightly (up to 12-14% w/w) as a result of the modification of paclitaxel molecule, e.g. through the attachment of hydrocarbon chains (US patent description Nos. 5,919,815, 5,939,567, 6,118,011).

There is thus still a need to design pharmaceutical liposome formulations, particularly liposome formulations containing a hydrophobic anti-tumour compounds, with a favourable lipid to active compound ratio, or ones facilitating the transfer of the same amount of active compound by a smaller quantity of lipid carrier.

The second unmet need is the delivery of a liposome containing two anti-tumour compounds for the combined therapy with an increased therapeutic effect, and characterised by a high stability.

Unexpectedly, the aforementioned problems have been solved by the present invention.

The first subject of the present invention is a liposome formulation containing an anti-tumour compound characterised in that it contains an active compound enclosed in liposome vesicles forming a composition of lipid components in the ratio of 1 part by mass of the active compound per 3 to 12 parts by mass of lipid components, preferably 1 part by mass of the active compound per 5 parts by mass of lipid components as well as a vitamin or a derivative thereof. Preferably, a liposome formulation according to the present invention is characterised in that as the active compound it contains anthracyclines. Preferably, a liposome formulation according to the present invention is characterised in that the vitamin is ascorbic acid or a salt thereof, preferably ammonium ascorbate, folic acid or a salt thereof, preferably ammonium folate or pantothenic acid, preferably an ammonium salt thereof. Equally preferably, a liposome formulation according to the present invention is characterised in that vitamin derivative is methothrexate.

The second subject of the present invention is a method of producing liposome formulation with an anti-tumour active compound, characterised in that encompasses
a) the formulation of unilamellar liposomes through the hydration of a phospholipid mixture, preferably hydrogenated soya or egg lecithin or distearylphosphatidylcholine as well as cholesterol, preferably in an amount from 30 mg/ml to 60 mg/ml in a 300mM solution of a vitamin or its derivative, preferably an ammonium salt of ascorbic or folic or pantothenic acid;
b) freezing and thawing of the suspension of liposomes in a solution of vitamin salt or its derivative, in liquid nitrogen and warm water, with a temperature above the phase transition temperature of the main lipid, in order to equalise the concentrations of vitamin salt or its derivative on both sides of the liposome bilayers;
c) decreasing the size and layers of liposomes, preferably through extruding the liposomes through polycarbonate filters with appropriate pore size in a high pressure extruder, or through homogenizing in a high-pressure homogenizer;
d) exchanging the external solution of the liposomes, preferably through dialysis or molecular sieving, to a neutral buffer, preferably PBS in order to form a pH and ionic gradient;
e) addition of the anti-tumour active compound, preferably anthracycline;
f) heating the suspension to a temperature higher than the phase transition temperature of the main phospholipid forming the liposomes, preferably to 60 °C.

Equally preferably, a method according to the present invention is characterised in that during stage a) PEGylated phosphatidylethanolamine is added in order to stabilize the liposomes in the blood circulation system. Preferably a method according to the present invention is characterised in that the vitamin derivative used in stage a) is methothrexate or an ammonium salt thereof.

The third subject of the present invention is a pharmaceutical composition for parenteral administration encompassing a pharmaceutically permissible carrier and/or auxiliary compounds and a therapeutically effective amount of anti-tumour active compound, characterised in that it contains a liposome formulation containing an anti-tumour compound enclosed in liposome vesicles forming the composition of lipid components in the ratio of 1 part by mass of the active compound per 3 to 12 parts by mass of the lipid components, preferably 1 part by mass of the active compound per 5 parts by mass of the lipid components as well as a vitamin or a derivative thereof. Equally preferably, a composition according to the present invention is characterised in that it contains methothrexate or a salt thereof as well as a compound from among the anthracyclines.

A liposome according to the present invention is characterised by a desirable lipid/active compound ratio as well as a high encapsulation efficiency of the drug in liposomes after 5-10 minutes of the encapsulation process according to the method presented in the description. Additionally, a method according to the present invention makes it possible to prepare liposomes for the so-called combined therapy, where two different drugs are used in order to increase the therapeutic effect. Thus, in this case, one drug is used to encapsulate the second, obtaining as a consequence two drugs enclosed in the structure of a single liposome. This procedure additionally makes it possible to decrease the leakage of the vitamin derivative complex, e.g. methothrexate and anthracyclines, which stabilizes both drugs in the liposomes. In the case of methothrexate by itself, its leakage following intravenous administration may be too rapid due to the presence of aqueous drug solution, whereas the precipitated form of the drug is favoured for its greater stability.

Embodiments of the present invention are shown in the figures, wherein Fig. 1 represents a graph of the kinetics of the encapsulation of Epirubicin in HSPC/Chol/DSPE-PEG 2000 5.5:4:0.5 mol/mol liposomes due to the use of an ion gradient of 300 mM of an ammonium salt of ascorbic acid. The conditions of drug encapsulation are given in Example 1. A-C; Fig. 2 effect of the drug:lipid ratio on the Epirubicin encapsulation efficiency in HSPC/Chol/DSPE-PEG 2000 5.5:4:0.5 mol/mol liposomes due to the use of an ion gradient of 300 mM of an ammonium salt of ascorbic acid. The conditions of drug encapsulation are described in Example 2 A; Fig. 3 is the retention of Epirubicin in HSPC/Chol/DSPE-PEG 2000 5.5:4:0.5 mol/mol liposomes encapsulated using an ion gradient of 300 mM of an ammonium salt of ascorbic acid. The conditions of encapsulating the drugs are described in Example 3. A. and Fig. 4 represents the Epirubicin encapsulation efficiency in HSPC/Chol/DSPE-PEG 2000 5.5:4:0.5 mol/mol liposomes using an ion gradient of 300 mM of an ammonium salt of respectively folic acid, pantothenic acid as well as methothrexate. The conditions of drug encapsulation are as shown in Example 4. A-D.

### Example 1

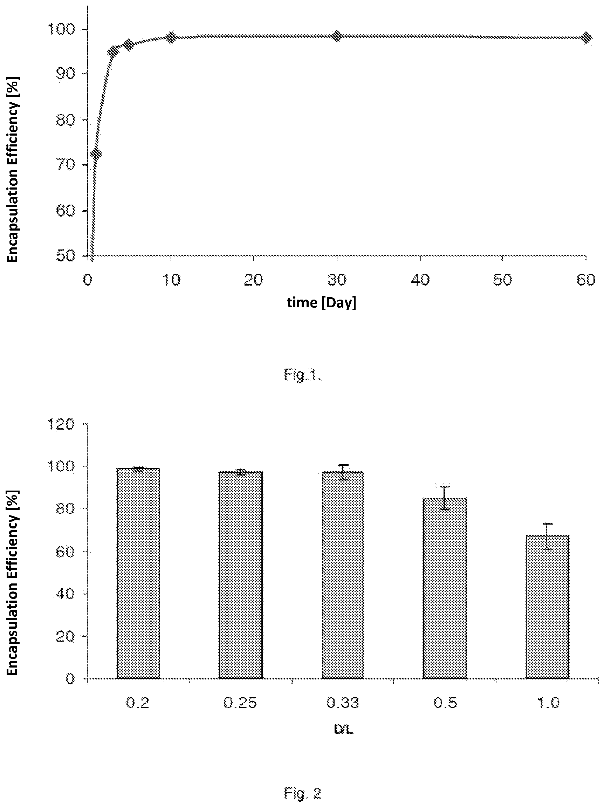

A. Production of liposomes containing ascorbic acid.
   A 100 ml round-bottomed flask was loaded with 35.28 mg hydrogenated soya lecithin (HSPC) and 13.02 mg cholesterol (Chol) as well as 11.67 mg PEGylated distearylphosphatidylethanolamine (DSPE-PEG 2000) in the form of chloroform solutions, and then organic solvent was evaporated using a vacuum evaporator. The dry lipid film was supplemented with a 1.5 ml solution of an ammonium salt of ascorbic acid (pH = 4,0) at a concentration of 300 mM, and then hydrated at a temperature of 64°C to the point when multilamellar liposomes were obtained.
   The liposome suspension was repeatedly frozen and thawed by alternating treating in liquid nitrogen and water with a temperature of 64°C, and then extruded in a pressure calibrator through a polycarbonate filter with a pore diameter of 100 nm at a temperature of 64°C.
B. Generation of an ion gradient of an ammonium salt of ascorbic acid.
   Liposomes obtained in pt. A were loaded on a column (1x20 cm) filled with a Sephadex G-50 fine gel, equilibrated in PBS, and then desalted, at the same time exchanging the external solution (300 mM solution of an ammonium salt of ascorbic acid) for phosphate buffer (PBS, pH =7,4). After separating the liposomes, the lipid concentration was determined using the Steward method.
C. Encapsulation of Epirubicin in HSPC/Chol/DSPE-PEG 2000 5.5:4:0.5 mol/mol liposomes.
   A suspension of liposomes comprising HSPC/Chol/DSPE-PEG 2000 5.5:4:0.5 mol/mol containing 300 mM of ascorbic acid ammonium salt were supplemented with a solution of Epirubicin hydrochloride in 150 mM of NaCl at a concentration of 6 mg/ml in such an amount so that per 5 parts by mass of the lipid there was one part by mass of the drug. The suspension was mixed and heated for 60 minutes at a temperature of 60 °C collecting suspension samples at selected time intervals. Following incubation, we determined the encapsulation efficiency of the drug, which after 10 minutes was 98%. The experimental conditions were set so that the final lipid concentration was 15 mg/ml. A detailed description of the drug encapsulation is shown in Fig. 1

### Example 2.

A. The effect of the drug lipid ratio on the encapsulation efficiency of Epirubicin in HSPC/Chol/DSPE-PEG 2000 5.5:4:0.5 mol/mol liposomes due to the use of an ion gradient 300 mM of an ammonium salt of ascorbic acid.

Liposomes were prepared as in Example 1. A-C, with the difference that the process of drug encapsulation was performed for several drug/lipid mass ratios; 1:5 (0.2) to 1:1 (1.0) . The time of drug encapsulation in each case was set at 10 minutes in 60 °C. After each incubation step, the liposomes were separated from unecapsulated drug in order to determine the encapsulation efficiency of the drug. The results obtained are shown in Fig. 2.

### Example 3

A. Effect of storage time of liposomes in 4 °C on Epirubicin retention in HSPC/Chol/DSPE-PEG 2000 5.5:4:0.5 mol/mol liposomes in which the drug was encapsulated using an ion gradient of 300 mM of an ammonium salt of ascorbic acid.

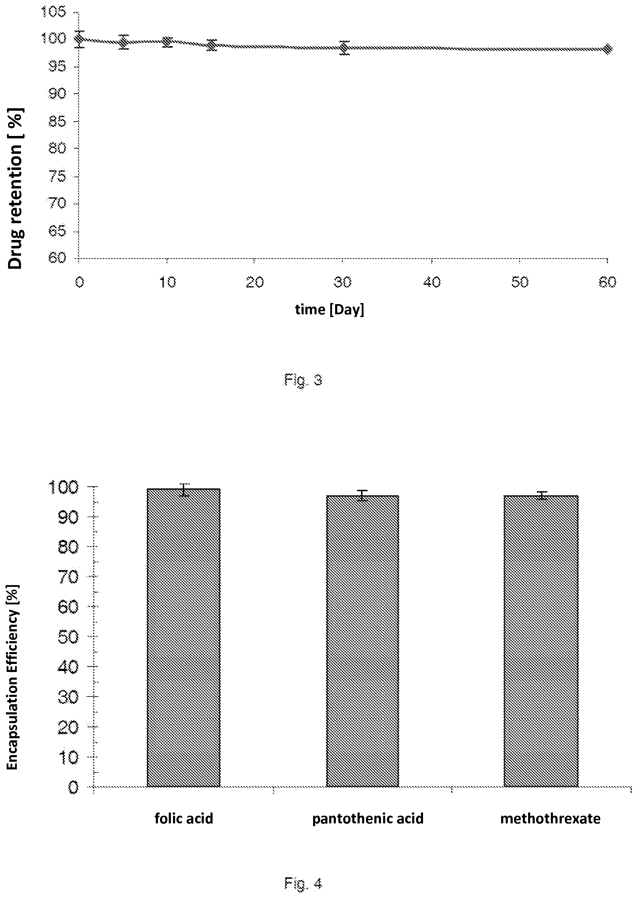

Liposomes obtained as in Example 1 were incubated for 10 min in order to encapsulate the drug and then unencapsulated drug was separated from the liposomes on a mini column of 0.5X 7 cm filled with a Sephadex G-50 fine gel. The separated liposomes were collected and then, following the determination of the amount of lipid this was diluted with PBS buffer pH = 7,4 such that the final lipid concentration was 10 mg/ml. Liposomes were stored at 4 °C. From the resulting portions of liposomes, at selected intervals, were collected 50 µl samples of liposomes and then were separated in order to determine the amounts of freed drug. Data obtained are shown in Fig. 3.

### Example 4.

A. Production of liposomes containing an ammonium salt of folic acid, pantothenic as well as methothrexate.
   Liposomes were prepared as in Example 1. A-B with the difference that instead of a 300 mM solution of an ammonium salt of ascorbic acid, pH = 4.0 we used a 300 mM solution of an ammonium salt of folic acid, pH = 7.8 or a 300 mM solution of an ammonium salt of acid pantothenic acid pH = 7.8 or a 300 mM solution of an ammonium salt of methothrexate.
B. Encapsulation of Epirubicin in HSPC/Chol/DSPE-PEG 2000 5.5:4:0.5 mol/mol liposomes using an ion gradient of an ammonium salt of folic acid.
   A suspension of liposomes comprising HSPC/Chol/DSPE-PEG 2000 5.5:4:0.5 mol/mol containing a 300 mM ammonium salt of folic acid was supplemented with a solution of Epirubicin hydrochloride in 150 mM of NaCl at a concentration of 6 mg/ml, in such an amount so that for 5 parts by mass of the lipid there was one part by mass of the drug. The suspension was mixed and heated for 10 minutes at a temperature of 60°C. Following incubation we determined the efficiency of drug encapsulation, which was 99%. The experimental conditions were set so that the final lipid concentration was 15 mg/ml. A detailed description of the drug encapsulation process is shown in Fig. 4.
C. Encapsulation of Epirubicin in HSPC/Chol/DSPE-PEG 2000 5.5:4:0.5 mol/mol liposomes using an ion gradient of an ammonium salt of pantothenic acid.
   Encapsulation of drug in liposomes was performed as in Example 4. B, with the difference that the liposomes contained not 300 mM of folic acid ammonium salt pH = 7.8, but 300 mM ammonium salt of pantothenic acid. The efficiency of drug encapsulation was 97%.
D. Encapsulation of Epirubicin in HSPC/Chol/DSPE-PEG 2000 5.5:4:0.5 mol/mol liposomes using an ion gradient of 300 mM of an ammonium salt of methothrexate pH =7.8.

Encapsulation of drug in liposomes was performed as in Example 4. B with the exception that the liposomes contained not 300 mM folic acid ammonium salt pH = 7.8, but 300 mM of methothrexate ammonium salt. The drug encapsulation efficiency was 97%.

The encapsulation results obtained in points B-D, for 300 mM salts of folic acid, pantothenic as well as methothrexate are shown in Fig. 4.

## Claims

1. A liposomal formulation containing an epirubicin **characterised in that** it contains an epirubicin in liposome comprising HSPC/Chol/ DSPE-PEG 2000 5.5:4:0.5 mol/mol vesicles forming a composition of lipid components in the ratio of 1 part by mass of the epirubicin per 3 to 12 parts by mass of the lipid components, and a 300mM solution of ascorbic acid or a salt thereof, preferably ammonium ascorbate or 300mM solution of folic acid or a salt thereof, preferably ammonium folate or 300mM solution of pantothenic acid, preferably an ammonium salt thereof or 300mM solution of methothrexate or an ammonium salt thereof.

2. A method of producing the liposome formulation of claim 1, **characterised in that** it encompasses
a) formulation of multilamellar liposomes through the hydration of a mixture of phospholipids, preferably hydrogenated soya or egg lecithin or distearylphosphatidylcholine as well as cholesterol, preferably in an amount from 30 mg/ml to 60 mg/ ml in a 300mM solution of ascorbic acid or pantothenic acid or folic acid or methothrexate or its derivative, preferably an ammonium salt of ascorbic or folic, pantothenic acid or methothrexate , preferably PEGylated phosphatidylethanolamine is added in order to stabilize the liposomes in the blood circulation system
b) freezing and thawing of the suspension of liposomes in a solution of ascorbic acid or a salt thereof, preferably ammonium ascorbate, folic acid or a salt thereof, preferably ammonium folate or pantothenic acid, preferably an ammonium salt thereof and methothrexate or an ammonium salt thereof in liquid nitrogen and warm water, with a temperature above the phase transfer temperature of the main lipid, in order to equilibrate the concentrations of vitamin salt or its derivative on both sides of the liposome membranes;
c) decreasing the size and layer counts of the liposomes, preferably by extruding the liposomes through polycarbonate filters of appropriate pore sizes in a high pressure extruder, or homogenizing in a high pressure homogenizer;
d) exchanging the external solution of the liposomes, preferably through dialysis or molecular sieving, for a neutral buffer, preferably PBS in order to create the pH/ionic gradient;
e) adding epirubicin;
f) heating the suspension to a temperature greater than the phase transition temperature of the main phospholipid forming the liposomes, preferably to 60 °C.

3. A pharmaceutical composition for parenteral administration encompassing a pharmaceutically permissible carrier and/or auxiliary compounds and a therapeutically effective amount of epirubicin, **characterised in that** it contains a liposomal formulation containing an epirubicine enclosed in liposome comprising HSPC/Chol/ DSPE-PEG 2000 5.5:4:0.5 mol/mol vesicles forming a composition of lipid components in the ratio of 1 part by mass of the anthracycline per 3 to 12 parts by mass of the lipid components, and 300 mM solution of ascorbic acid or a salt thereof, preferably ammonium ascorbate, 300 mM solution of folic acid or a salt thereof, preferably ammonium folate or 300 mM solution of pantothenic acid, preferably an ammonium salt thereof or 300 mM solution of methothrexate or an ammonium salt thereof.

## Patentansprüche

1. Liposomale Formulierung enthaltend ein Epirubicin, **dadurch gekennzeichnet, dass** sie ein Epirubicin in Liposom enthält, umfassend 5,5:4:0,5 Mol/Mol HSPC/Chol/DSPE-PEG 2000-Vesikel, die eine Zusammensetzung von Lipidkomponenten in dem Verhältnis von 1 Masseteil Epirubicin pro 3 bis 12 Masseteile der Lipidkomponenten bilden, und eine 300 mM Lösung von Ascorbinsäure oder einem Salz davon, bevorzugt Ammoniumascorbat oder 300 mM Lösung von Folsäure oder einem Salz davon, bevorzugt Ammoniumfolat oder 300 mM Lösung von Pantothensäure, bevorzugt einem Ammoniumsalz davon, oder 300 mM Lösung von Methothrexat oder einem Ammoniumsalz davon.

2. Verfahren für die Herstellung der Liposomformulierung nach Anspruch 1, **dadurch gekennzeichnet, dass** es Folgendes umfasst
a) Formulierung von multilamellaren Liposomen durch die Hydratisierung einer Mischung von Phospholipiden, bevorzugt von hydriertem Soja- oder Eilecithin oder Distearylphosphatidylcholin sowie Cholesterin, bevorzugt in einer Menge von 30 mg/ml bis 60 mg/ml in einer 300 mM Lösung von Ascorbinsäure oder Pantothensäure oder Folsäure oder Methothrexat oder seines Derivat, wobei bevorzugt ein Ammoniumsalz von Ascorbin- oder Fol-, Pantothensäure oder Methothrexat, bevorzugt PEGyliertem Phosphatidylethanolamin hinzugegeben wird, um die Liposome im Blutkreislaufsystem zu stabilisieren;
b) Gefrieren und Auftauen der Suspension von Liposomen in einer Lösung von Ascorbinsäure oder einem Salz davon, bevorzugt Ammoniumascorbat, Folsäure oder einem Salz davon, bevorzugt Ammoniumfolat oder Panthotensäure, bevorzugt einem Ammoniumsalz davon und Methothrexat oder einem Ammoniumsalz davon in flüssigem Stickstoff und warmem Wasser bei einer Temperatur über der Phasentransfertemperatur des Hauptlipids, um die Konzentrationen von Vitaminsalz oder seinem Derivat auf beiden Seiten der Liposommembranen zu äquilibrieren;
c) Reduzieren der Größe und Schichtzahlen der Liposome, bevorzugt durch Extrudieren der Liposome durch Polycarbonatfilter geeigneter Porengröße in einem Hochdruckextruder oder Homogenisieren in einem Hochdruckhomogenisator;
d) Austauschen der externen Lösung der Liposome, bevorzugt durch Dialyse oder Molekularsieben für einen neutralen Puffer, bevorzugt PRS, um den pH-Wert-/lonengradienten zu schaffen;
e) Zusetzen von Epirubicin;
f) Erhitzen der Suspension auf eine Temperatur höher als die Phasentransfertemperatur des Hauptphospholipids, das die Liposomen bildet, bevorzugt auf 60 °C.

3. Pharmazeutische Zusammensetzung zur parenteralen Verabreichung, umfassend einen pharmazeutisch zulässigen Träger und/oder Hilfsverbindungen und eine therapeutisch wirksame Menge Epirubicin,
**dadurch gekennzeichnet, dass** sie eine liposomale Formulierung enthält, die Epirubicin enthält, das in Liposom eingeschlossen ist, das 5,5:4:0,5 Mol/Mol HSPC/Chol/DSPE-PEG 2000-Vesikel umfasst, die eine Zusammensetzung von Lipidkomponenten im Verhältnis von 1 Masseteil des Anthracyclins pro 3 bis 12 Masseteile der Lipidkomponenten bilden, und 300 mM Lösung Ascorbinsäure oder eines Salzes davon, bevorzugt Ammoniumascorbat, 300 mM Lösung von Folsäure oder einem Salz davon, bevorzugt Ammoniumfolat oder 300 mM Lösung von Pantothensäure, bevorzugt Ammoniumsalz davon oder 300 mM Lösung von Methothrexat oder einem Ammoniumsalz davon.

## Revendications

1. Formulation liposomale contenant une épirubicine, **caractérisée par le fait qu'**elle contient une épirubicine dans des vésicules de liposomes comprenant HSPC/Chol/DSPE-PEG 2000 5,5:4:0,5 mol/mol formant une composition de composants lipidiques dans le rapport de 1 partie en masse de l'épirubicine pour 3 à 12 parties en masse des composants lipidiques, et une solution 300mM d'acide ascorbique ou d'un sel de celui-ci, de préférence l'ascorbate d'ammonium, ou une solution 300mM d'acide folique ou d'un sel de celui-ci, de préférence le folate d'ammonium, ou une solution 300mM d'acide pantothénique, de préférence un sel d'ammonium de celui-ci, ou une solution 300mM de méthotrexate ou d'un sel d'ammonium de celui-ci.

2. Procédé de production de la formulation de liposomes selon la revendication 1, **caractérisé par le fait qu'**il comprend :
a) formulation de liposomes multilamellaires par l'hydratation d'un mélange de phospholipides, de préférence la lécithine de soja ou d'oeuf hydrogénée ou la distéarylphosphatidylcholine ainsi que le cholestérol, de préférence dans une quantité de 30 mg/ml à 60 mg/ml dans une solution 300mM d'acide ascorbique ou d'acide pantothénique ou d'acide folique ou de méthotrexate ou son dérivé, de préférence un sel d'ammonium d'acide ascorbique ou folique, d'acide pantothénique ou de méthotrexate, de préférence de la phosphatidyléthanolamine PEGylée est ajoutée de façon à stabiliser les liposomes dans le système de la circulation sanguine ;
b) congélation et décongélation de la suspension de liposomes dans une solution d'acide ascorbique ou d'un sel de celui-ci, de préférence l'ascorbate d'ammonium, d'acide folique ou d'un sel de celui-ci, de préférence le folate d'ammonium, ou d'acide pantothénique, de préférence un sel d'ammonium de celui-ci et de méthotrexate ou d'un sel d'ammonium de celui-ci dans de l'azote liquide et de l'eau chaude, avec une température au-dessus de la température de transition de phase du lipide principal, de façon à équilibrer les concentrations de sel de vitamine ou de son dérivé des deux côtés des membranes de liposomes ;
c) diminuer la dimension et le nombre de couches des liposomes, de préférence par extrusion des liposomes à travers des filtres de polycarbonate de dimensions de pore appropriées dans une extrudeuse haute pression, ou par homogénéisation dans un homogénéiseur haute pression ;
d) échanger par la solution externe des liposomes, de préférence par dialyse ou tamisage moléculaire, un tampon neutre, de préférence le PBS de façon à créer le gradient de pH/ionique;
e) ajouter de l'épirubicine ;
f) chauffer la suspension à une température supérieure à la température de transition de phase du phospholipide principal formant les liposomes, de préférence à 60°C.

3. Composition pharmaceutique pour l'administration parentérale comprenant un support pharmaceutiquement acceptable et/ou des composés auxiliaires pharmaceutiquement acceptables et une quantité thérapeutiquement efficace d'épirubicine,
**caractérisée par le fait qu'**elle contient une formulation liposomale contenant une épirubicine enfermée dans des vésicules de liposomes comprenant HSPC/Chol/DSPE-PEG 2000 5,5:4:0,5 mol/mol formant une composition de composants lipidiques dans le rapport de 1 partie en masse de l'anthracycline pour 3 à 12 parties en masse des composants lipidiques, et une solution 300mM d'acide ascorbique ou d'un sel de celui-ci, de préférence l'ascorbate d'ammonium, ou une solution 300mM d'acide folique ou d'un sel de celui-ci, de préférence le folate d'ammonium, ou une solution 300mM d'acide pantothénique, de préférence un sel d'ammonium de celui-ci, ou une solution 300mM de méthotrexate ou d'un sel d'ammonium de celui-ci
